# EUROPEAN PATENT APPLICATION

(11) **EP 2 064 985 A2**
(43) Date of publication of application: **03.06.2009**
(21) Application number: 08020530.5
(22) Date of filing: 26.11.2008
(51) Int. Cl.: A61B 1/018, A61B 17/28

(54) **Apparatus for controlling endoscopic instrument**

(30) Priority: 27.11.2007 US 990376 P
(71) Applicant: Tyco Healthcare Group LP, North Haven, CT 06473 (US)
(72) Inventor: Bahney, Timothy J., Portland, Oregon 97209 (US)
(74) Representative: HOFFMANN EITLE

(57) **Abstract**

An apparatus for the control of endoscopic instruments includes a control module (16) mountable to a standard instrument port (32) of an endoscope (12). The control module includes separate drive mechanisms (60,64) for a sheath (46) and a tensile cord (48) of an endoscopic tool having an end effector (38). Driving the sheath and the tensile cord together may serve to advance or withdraw the end effector, driving either component separately may cause an activation of the end effector.

## Description

### CROSS-REFERENCE TO RELATED APPLICATIONS

This application claims the benefit of priority to U.S. Provisional Application Serial No. 60/990,376 entitled "METHOD AND APPARATUS FOR CONTROLLING ENDOSCOPIC INSTRUMENTS" filed November 27, 2007 by Timothy J. Bahney, which is incorporated by reference herein.

### BACKGROUND

### 1. Technical Field

The present disclosure relates generally to an apparatus and related method for performing endoscopic and endoluminal surgical procedures, and more specifically, to an apparatus and related method for controlling the operation of instrumentation commonly used in such procedures.

### 2. Background of Related Art

A surgeon will often need to cut tissue, occlude vessels or perform some other procedure at an operative site within a patient. Endoscopic and endoluminal procedures are increasingly preferred over traditional open procedures due to the attendant advantages including more expedient recoveries and a reduced risk of contaminating internal organs. In general, endoscopic and endoluminal surgeries involve the use of tube inserted into a patient through a small incision or a natural orifice. An optical system is inserted into the patient through the tube to allow a surgeon to view the interior environment of the patient. A surgical instrument is inserted through another tube to manipulate tissue. In many procedures the optical system is located in one lumen of a flexible endoscope having at least one other lumen available for the introduction of a variety of instruments that might be required for the surgery.

Endoscopic instruments commonly incorporate a flexible elongate tube coupled to a handle at the proximal end and an end effector at the distal end. The end effector may, for example, take the form of a forceps having a pair of jaws adapted to articulate between open and closed configurations to clamp or grasp tissue. The jaws may be biased to the open configuration and may be moved to the closed configuration by pulling a thumb ring located on the proximal handle. A tensile element inside the flexible elongate tube connects the thumb ring to the end effector to facilitate this motion.

In performing an endoscopic or endoluminal procedure, a physician may first insert the endoscope into a patient's body to identify the surgery site with the optical system. Once the site has been located, the physician may insert a forceps or other end effector through the available instrumentation lumen while still holding the endoscope to maintain proper positioning. Once the surgeon is satisfied with the positioning of both the end effector and endoscope, the surgeon must often rely on an assistant to pull the thumb ring and close the jaws so that the surgeon may focus sufficient attention and faculties to the positioning of the components. The need for at least two skilled practitioners complicates the surgery increasing the risk of miscommunication and errors.

### SUMMARY

The present disclosure describes an apparatus for performing a surgical procedure on a patient. The apparatus includes a tool having a hollow member and an actuation member extending through the hollow member. The hollow member and actuation member are coupled to an end effector such that a relative motion between the hollow member and the actuation member serves to actuate the end effector. A main driver is operatively connected to the tubular member and adapted to effect a longitudinal motion of the tubular member. An independent follower driver is operatively coupled to the actuation member and adapted to independently effect a longitudinal motion of the actuation member.

The main driver and follower driver may be mounted in a tool control module mountable to an instrument port of an endoscope. A user interface may be disposed on a body portion of the endoscope for accepting instructions from a user to be transmitted to the main driver and follower driver.

The tubular member may comprise a sheath having a longitudinal seam such that an opening may be maintained where the actuation member may diverge from the sheath. A coupling member near the distal end of the tubular member may be included for permitting selective attachment and removal of the end effector from the surgical tool. The end effector may be configured for delivering electrosurgical energy to the the tissue.

The tool control module may also be adapted for rotation relative to the instrument port of the endoscope. Such a rotation may effect a complementary rotation in the end effector.

Another embodiment of the disclosure involves an endoscopic tool control system. The system includes an endoscope having a distal end configured for positioning within a body cavity of a patient, an instrument port near a proximal end of the endoscope and an instrumentation lumen extending from the instrument port to the distal end of the endoscope. The system further includes an endoscopic tool partially positionable within the instrumentation lumen. The endoscopic tool includes an elongate tubular member and an elongate actuation member extending proximally from an end effector such that a relative motion of the actuation member with respect to the tubular member effects an actuation or activation of the end effector. The system further includes a tool control module coupled to the instrument port of the endoscope. The tool control module includes a main driver operatively connected to the tubular member and adapted for selectively imparting a longitudinal motion to the tubular member, and a follower driver operatively connected to the actuation member and adapted for selectively imparting an independent longitudinal motion to the actuation member.

The control system may include a user interface disposed on a body portion of the endoscope for accepting instructions from a clinician to effect a motion in the end effector. The tubular member may include a longitudinal seam allowing an opening to be maintained on a longitudinal side of the tubular member to permit the actuation member to pass from an interior side of the tubular member to an exterior side of the tubular member. A coupling member may be included for permitting selective attachment and removal of the end effector from the endoscopic tool. The end effector may take the form of an electrosurgical forceps assembly or a snare loop assembly.

A method is also described for controlling an endoscopic tool. The method includes the steps of providing a tool having an end effector coupled to a distal end of a sheath and and a distal end of an actuation member such that the end effector is activated by imparting a relative motion to the actuation member with respect to the sheath, providing an endoscope having a tool control module coupled to an instrument port thereof wherein the tool control module includes a first drive mechanism adapted for selectively driving the sheath and a second drive mechanism for independently driving the actuation member and a user interface on a body portion of the endoscope having control surfaces in communication with the first and second drive mechanisms, grasping the body portion of the endoscope to position a distal end of the endoscope in proximity to a target tissue, positioning the end effector in proximity to the target tissue by activating at least one of the control surfaces, and activating the end effector by activating another of the control surfaces.

The step of positioning the end effector may be accomplished by contacting two control surfaces simultaneously and the step of actuating the end effector may be accomplished by contacting only a single one of the control surfaces. The method may further include a step of orienting the end effector by rotating the tool control module.

### BRIEF DESCRIPTION OF THE DRAWINGS

The accompanying drawings, which are incorporated in and constitute a part of this specification, illustrate embodiments of the present disclosure and, together with the detailed description of the embodiments given below, serve to explain the principles of the disclosure.
FIG. 1 is a perspective view of an illustrative embodiment of a remotely controlled endoscopic tool system of the present the disclosure;
FIG. 2 is a schematic representation of the system of FIG. 1;
FIG. 3A through FIG 3C are perspective views of various configurations for a portion of a catheter sheath having a longitudinal seam and an actuation member for use with the surgical tool of FIG. 1;
FIG. 4A through 4C are top side views of alternate end effectors for use with the surgical tool of FIG. 1;
FIG. 5 is a schematic representation of an alternate illustrative embodiment of an endoscopic tool system of the present disclosure; and
FIG. 6 is a schematic representation of another alternate illustrative embodiment of an endoscopic tool system of the present disclosure.

### DETAILED DESCRIPTION

The attached figures illustrate exemplary embodiments of the present disclosure and are referenced to describe the embodiments depicted therein. Hereinafter, the disclosure will be described in detail by explaining the figures wherein like reference numerals represent like parts throughout the several views. In the drawings and in the description that follows, the term "proximal," as is traditional, will refer to the direction toward the operator or a relative position on the surgical device or instrument that is closer to the operator, while the term "distal" will refer to the direction away from the operator or relative position of the instrument that is further from the operator.

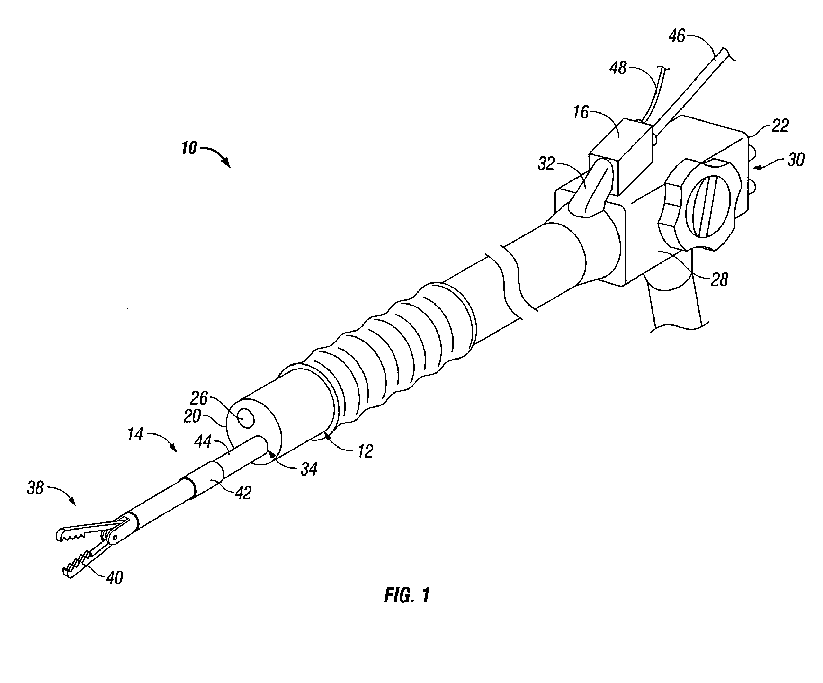

Referring initially to FIG. 1, an embodiment of a remotely operated endoscopic tool system 10 generally includes endoscope 12, endoscopic tool 14, and tool control module 16 as shown. A distal end 20 of endoscope 12 is adapted for insertion into an interior lumen or body cavity of a patient while a proximal end 22 of endoscope 12 is adapted to remain on the exterior of the patient to be handled by a clinician. As used herein, the term clinician refers to a surgeon, doctor, nurse, or other care provider and may include support personnel.

At the distal end 20 of endoscope 12, a viewing window 26 shields an optical system (not shown) internal to the endoscope 12. The optical system may provide a camera, illumination device, or other suitable equipment to permit the clinician to view the environment exterior to the distal end 20 of the endoscope 12. The proximal end 22 of the endoscope 12 includes a body 28 having a user interface 30, which allows the clinician to control the endoscopic tool 14 as discussed below. Extending from the body 28 is an instrument port 32 providing access to an internal instrumentation lumen 34 running the length of the endoscope 12.

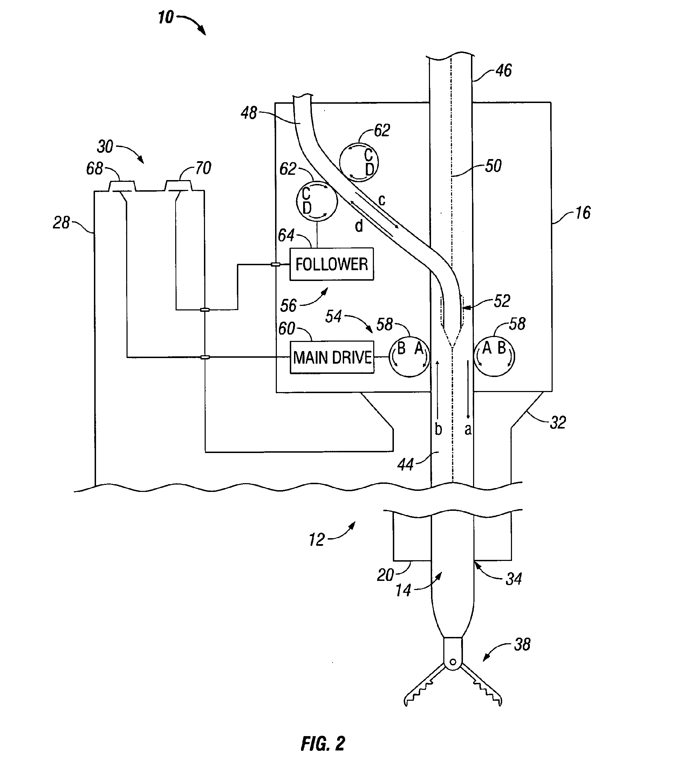

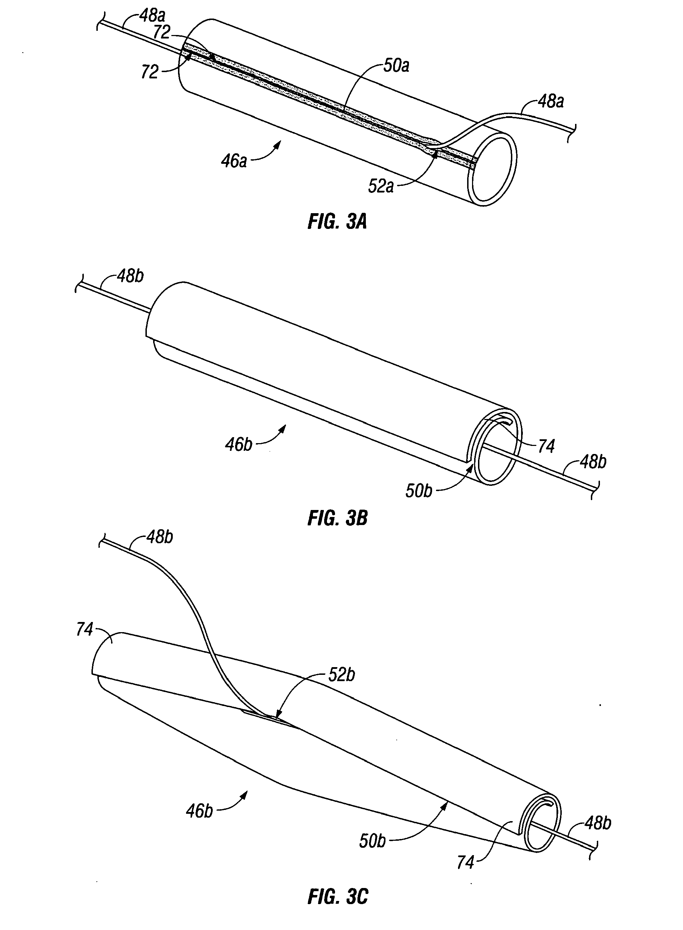

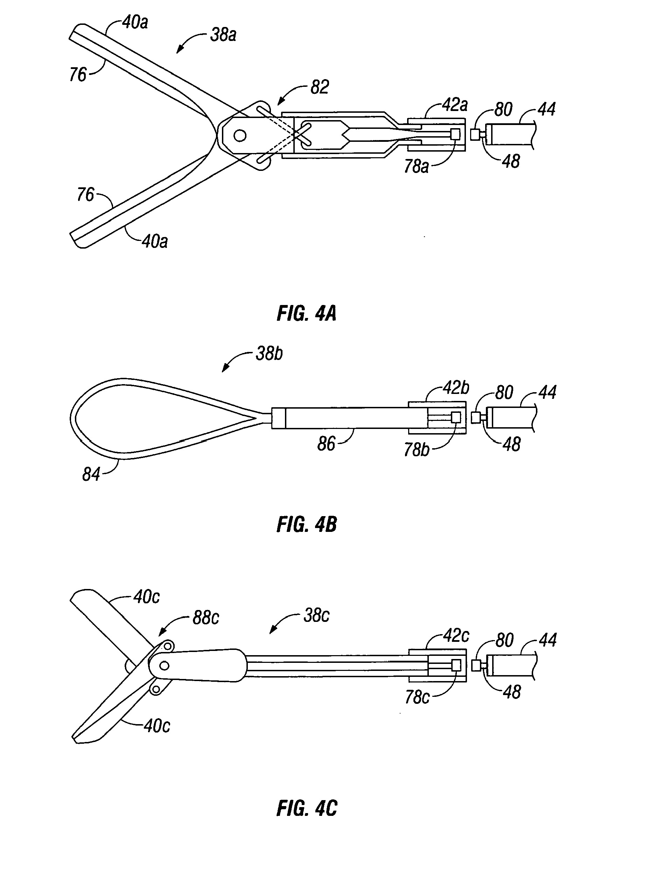

Instrumentation lumen 34 receives endoscopic tool 14 permitting both longitudinal and rotational movement of endoscopic tool 14 therein. In the illustrated embodiment, endoscopic tool 14 generally includes an end effector 38 having a pair of articulating jaw members 40, a coupling member 42, and a catheter 44. End effector 38 is adapted to manipulate tissue at a surgery site within the body cavity or lumen, here taking the form of a grasper for holding tissue between articulating jaw members 40. Other forms for an end effector are envisioned as discussed hereinbelow with reference to FIG. 4A through 4C. Coupling member 42 releasably connects end effector 38 to a catheter 44 such that a variety of end effectors may be exchanged when required. Catheter 44 comprises a hollow tubular member, such as flexible sheath 46, and an actuation member, such as tensile cord 48. Sheath 46 radially surrounds the tensile cord 48 near the distal end 20 and for most of the length of catheter 44. The sheath 46 and the tensile cord 48 may diverge, as discussed below, in the tool control module 16 such that each may exit the control module 16 separately as shown. A longitudinal seam 50 (FIG. 2) may be formed in at least a portion of the sheath 46 to permit such a divergence of the sheath 46 and tensile cord 48. Alternative embodiments for longitudinal seam 50 are discussed in greater detail hereinbelow with reference to FIG 3A to 3C.

Sheath 46 and tensile cord 48 are connected to the end effector 38 such that a relative motion of the tensile cord 48 with respect to the sheath 46 causes an activation of the end effector 38. For example, a proximal motion of the tensile cord 48 through a stationary sheath 46 may impart a tensile force on tensile cord 48, thereby causing an approximation or closing of the articulating jaw members 40. Similarly, a distal motion of tensile cord 48 with respect to sheath 46 may relax a tension in tensile cord 48 allowing jaw members 40 to open. A combined longitudinal motion of the tensile member 48 and the sheath 46, however, does not activate the end effector 38. This type of combined motion permits the end effector 38 to be advanced (distally) or withdrawn (proximally) with respect to the instrumentation lumen 34 of the endoscope 12. A combined rotation of the sheath 46 and tensile cord 48 can adjust the orientation of the end effector 38 with respect to the endoscope 12. Control module 16 may be adapted to impart these and other motions to the end effector as discussed below.

Figure 2 depicts a schematic representation of the remotely operated endoscopic tool system 10. Control module 16 is mounted to the instrument port 32 of endoscope 12. The catheter 44 of endoscopic tool 14 is routed through the control module 16 and instrument port 32 into the internal instrumentation lumen 34. The sheath 46 of catheter 44 includes a longitudinal seam 50 along at least a portion of its length. An opening 52 in the longitudinal seam 50 is maintained on the interior of control module 16. The opening 52 defines a juncture where the sheath 46 and the tensile cord 48 diverge. Proximally of the juncture, the two components are separated permitting each to be manipulated individually. Distally of the juncture, the tensile cord 48 runs co-axially through the sheath 46. The sheath 46 and the tensile cord 48 may protrude separately from the proximal side of control module 16.

Mounted on the interior of the control module 16 are two independent drive mechanisms 54, 56 for driving the tensile cord 48 and the sheath 46. A first drive mechanism 54 may comprise a first set of friction drive wheels 58 in frictional contact with the outer sheath 46, and a main driver 60 operatively connected to the drive wheels 58. The main driver 60 may comprise a motor, such as a step motor or servo motor, and serves to rotate friction drive wheels 58 in opposite directions such that the drive wheels 58 cooperate to drive the sheath 46 in a longitudinal direction. For example, rotation of drive wheels 58 in the direction of arrows "A" tends to drive the sheath 46 distally in the direction of arrow "a", and rotation of drive wheels 58 in the direction of arrows "B" tends to drive the sheath 46 proximally in the direction of arrow "b". Additionally, if drive wheels 58 are prevented from rotating, sheath 46 is prevented from moving due to the frictional contact between the drive wheels 58 and sheath 46. The first set of friction drive wheels 58 may be disposed distally of the juncture as shown, or in any other suitable location for driving the sheath 46.

A second drive mechanism 56 may comprise a second set of friction drive wheels 62 in frictional contact with tensile cord 48, and a follower driver 64 operatively connected to the second set of friction drive wheels 62. Follower driver 64 may comprise a step motor, servo motor or any suitable mechanism for driving drive wheels 62. The second drive mechanism 56 serves to drive the tensile cord 48 independently from sheath 46, but in a manner similar to the manner in which the first drive mechanism 54 drives the sheath 46. Rotation of the second set of drive wheels 62 in the direction of arrows "C" serves to drive the tensile cord distally in the direction of arrow "c", and rotation of the second set of drive wheels 62 in the direction of arrows "D" serves to drive the tensile cord proximally in the direction of arrow "d". As suggested above, main driver 60 and the follower driver 64 may serve to maintain the position of sheath 46 and tensile cord 48 by preventing rotation of their respective set of drive wheels 58, 62.

Both the main driver 60 and follower 64 are adapted to receive instructions from a remote user interface 30 having control surfaces such as a pair of buttons 68, 70 disposed on the body 28 of endoscope 12. User interface 30 may include other control surfaces, such as knobs or switches, in communication with the main driver 60 and follower 64 to enable a user to selectively drive both the sheath 46 and the tensile cord 48 according to a predetermined instructional scenario. For example, activating a single control surface, such as depressing button 68, may enable a user to drive the sheath 46 independently. Individually activating another control surface, such as depressing button 70 may enable a user to drive tensile cord 48 independently. In this case, depressing both buttons 68, 70 simultaneously may enable a user to drive both the sheath 46 and tensile cord 48 together. In this way, the clinician may depress a single button, 68 or 70 to create a relative motion between the sheath 46 and tensile cord 48 to activate end effector 38 as discussed above. The clinician may depress both buttons 68, 70 simultaneously to create a combined motion resulting in a longitudinal motion of end effector 38.

In an exemplary use, a clinician may grasp the body 28 of endoscope 12 to position the distal end 20 within a body cavity or a lumen of a patient. The clinician may use the on-board optical system to verify proper positioning. After achieving a satisfactory view of the body tissue to be manipulated, the clinician may advance the endoscopic tool 14 longitudinally though instrumentation lumen 34 by depressing both buttons 68, 70 simultaneously to drive both the sheath 46 and tensile cord 48 together to bring the end effector 38 within reach of the tissue to be manipulated. At this point, the clinician may release button 70 while continuing to depress button 68 to advance only sheath 46 individually while tensile cord 48 is held in position by friction drive wheels 62. This differential motion between the sheath 46 and the tensile cord 48 effectively pulls the tensile cord 48 to close the end effector 38 around the tissue. In this way, the clinician may maintain his grasp on the endoscope body 28 and manipulate tissue without relying on an assistant.

Other instructional scenarios are possible. For example, the user interface 30 may be configured such that activating a single control surface may enable a user to cause both the first and second drive mechanisms 54, 56 to drive the sheath 46 and the tensile cord 48 together to adjust the position of the end effector. Once the end effector 38 is in position, activating another control surface may open or close the end effector 38.

Referring now to FIGs. 3A through 3C, multiple embodiments of a sheath having a longitudinal seam are depicted for use with the present disclosure. As depicted in FIG. 3A, sheath 46a includes two abutment surfaces 72 that contact one another along longitudinal seam 50a. An opening 52a may be created along a portion of longitudinal seam 50a to allow tensile cord 48a to be withdrawn through a lateral side of the sheath 46a. The sheath 46a may be formed from a shape memory plastic that gives the sheath 46a a tendency to assume a closed configuration on either side of opening 52a. Alternatively, a closure structure including magnets or similar device may be employed.

The overlapping sheath 46b of FIG. 2B and 2C is fabricated from a semi-rigid plastic or another shape memory material that allows the sheath to assume an overlapping configuration in an unstressed state. The longitudinal seam 50b is non-joined, but the inherent memory in the material maintains a snug interface between overlap 74 and the exterior surface. In the unstressed state, overlapping sheath 46b may completely surround tensile element 48b as seen in FIG. 3B. Introducing an internal force directed outward, however, causes overlapping sheath 46b expand radially until the diameter is too large to maintain the overlap, and a lateral opening 52b is created. As seen in FIG. 3C, such an internal force may be locally applied by the tensile element 48b as it is withdrawn through the lateral opening 52b in the sheath 46b. The memory of the material causes the sheath 46b to close on either side of opening 52b. This feature allows the opening 52b to effectively translate along the length of the longitudinal seam 50b.

Referring now to FIGS. 4A through 4C, alternate embodiments of an end effector are described for use with the present disclosure. FIG. 4A depicts an end effector 38a comprising an electrosurgical forceps assembly of the type commonly used to cauterize, coagulate or seal tissue. End effector 38a includes a pair of articulating jaws 40a adapted move between an open position as shown and a closed position whereby tissue may be clamped between two sealing surfaces 76. A clinician may use the control surfaces 68, 70 (FIG. 2) of user interface 30 to position end effector 38a and clamp tissue as described above. An additional control surface (not shown) may be incorporated into user interface 30 to allow a clinician to selectively energize sealing surfaces 76 to deliver monopolar, bipolar or both types of electrosurgical energy to the tissue clamped between jaw members 40a.

Coupling member 42a connects end effector 38a to a distal end of the catheter 44. Coupling member 42a includes a connecting member 78a that corresponds to a connecting member 80 on the distal end of tensile cord 48c. The connecting members 78a, 80 may comprise a hook and loop interface or any other arrangement to allow a tensile force to be transmitted from tensile cord 48 to the scissor mechanism 82, which serves to close jaw members 40c. Connecting members 78a, 80 may also include an electrical interface suitable to deliver electrosurgical energy to jaw members 40a. Connecting members 78a, 80 may also be made standard to allow an assortment of end effectors to interface with catheter 44.

Referring now to FIG. 4B, end effector 38b takes the form of a snare loop of the type commonly used to resect an abnormal protruding growth. End effector 38b includes a loop portion 84 that may be positioned around tissue such as a polyp that a clinician wishes to remove. The polyp may be severed as a clinician activates end effector 38b to withdraw loop portion 84 into sheath portion 86 so that loop portion 84 closes tightly around the polyp. Loop portion 84 may be formed from an electrically conductive material so that electrosurgical energy may be delivered to the tissue as it is severed in order to help minimize bleeding, for example.

Because end effector 38b is activated in the same manner as end effector 38a, i.e. by providing a tensile force, coupling member 42b may operate in the same manner as coupling member 42a as described above. Coupling member 42b includes connecting member 78b for interfacing with the connecting member 80 at the distal end of tensile cord 48. Connecting members 78b and 80 may also include an electrical interface allowing electrosurgical energy to be transmitted to end effector 38b. As may be appreciated from the above description, the similarities between end effectors 38a and 38b may permit a similar operation involving the use of user interface 30 as previously described.

Referring now to FIG. 4C, end effector 38c takes the form of a scissors assembly adapted to cut tissue as jaw members 40c are closed. Once again, a jaw closure mechanism 88c may be provided to close jaws 40c upon application of a tensile force to through coupling member 42c including connecting member 78c. In this way, end effector 38c may be adapted to be exchanged with other end effectors, e.g. 38a, 38b, for use with a modular endoscopic tool system 10. Of course, other mechanisms (not shown) may be envisioned for closing jaw members or otherwise activating an end effector that respond to a differential motion between a sheath 46 and an activation member 48. For instance, a mechanism may be adapted to operate upon application of a tensile force to the sheath member 46, rather than an activation member 48. Although various components may need to be modified, such a mechanism is envisioned within the scope of this disclosure.

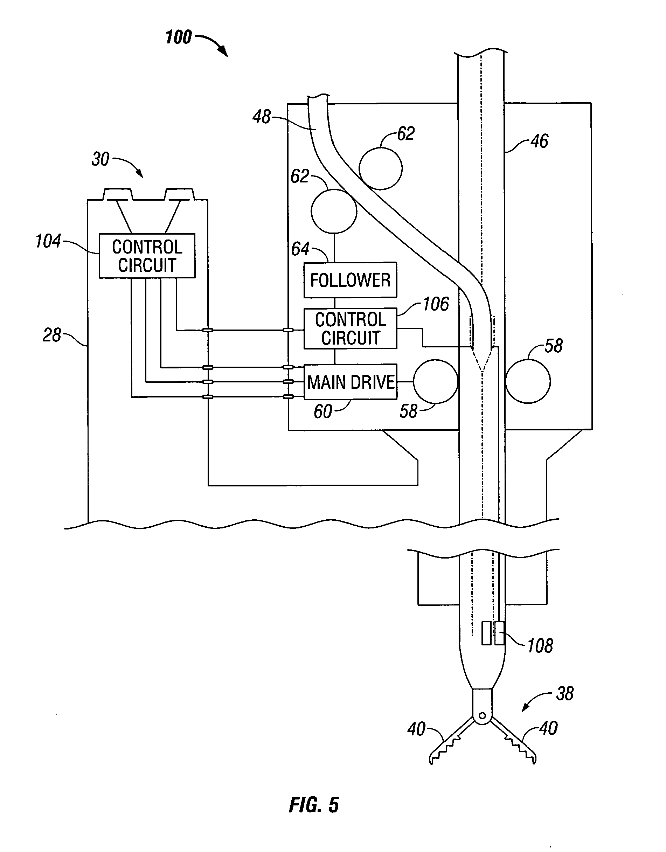

Referring now to FIG. 5, a schematic representation of another embodiment of a remotely operated endoscopic tool system is depicted generally as 100. As in the embodiment discussed with reference to FIG. 2, endoscopic tool system 100 includes two sets of friction wheels 58, 62 for driving sheath 46 and tensile cord 48 respectively for advancing and activating end effector 38. Main driver 60 and follower 64 are adapted to receive instructions from user interface 30 disposed on endoscope body 28.

Endoscopic tool system 100 further includes control circuits 104, 106 and sensor 108 to assist the clinician in controlling the ensdoscopic tool 14. Control circuit 104 is adapted to receive instructions from user interface 30 and transmit appropriate instructions for main drive 60 and follower 64 to accomplish the motion intended by the clinician. A feedback loop comprising sensor 108 and control circuit 106 provides additional information to assist in arriving at the proper instructions to transmit. Sensor 108 may be adapted to determine when a particular relative position between the sheath 46 and tensile cord 48 has been achieved. For example, sensor 108 may transmit a signal to control circuit 106 to indicate that sheath 46 and tensile cord 48 are relatively positioned such that jaw 40 of end effector 38 are in a fully open state.

Control circuit 106 may process this information from sensor 108 together with instructions received from control circuit 104, and any information available from main driver 60, to deliver appropriate instructions to main driver 60 and follower 64. Control circuits 104, 106 and sensor 108 may assist a clinician by adapting the instructions transmitted to the particular type of tissue encountered. Additionally, these features may assist a clinician in achieving compound motions in the end effector 38.

One example of a compound motion involves the use of a snare loop end effector 38b as discussed with reference to FIG. 4B. A clinician may need to maintain a relative position of tissue captured in a particular region of the loop portion 84. Because the relative position of tissue captured might otherwise change as loop portion 84 is drawn closed, it may be helpful to advance sheath 46 distally while simultaneously retracting tensile cord 48 proximally. This can help to prevent tissue from being drawn into sheath portion 86 as loop portion 84 closes, for example. Components such as control circuits 104, 106 and sensor 108 may allow a clinician to send a single instruction from a user interface 30 (FIG. 2) to produce such a compound motion.

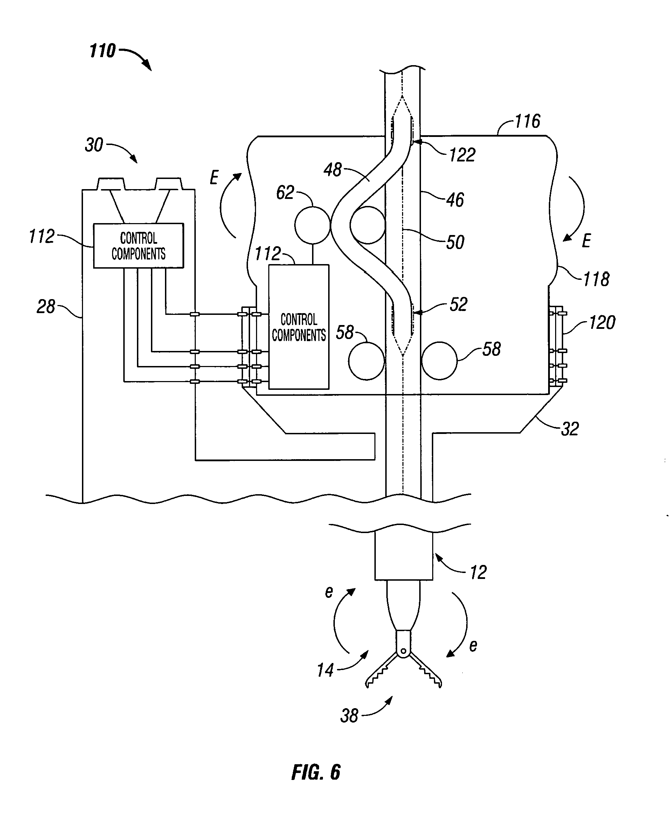

Referring now to FIG. 6, a schematic representation of another embodiment of a remotely operated endoscopic tool system is depicted generally as 110. Endoscopic tool system 110 permits a rotation of endoscopic tool 14 to allow a user to adjust the orientation of end effector 38. As in the embodiment discussed with reference to FIG. 2, endoscopic tool system 110 includes two sets of friction wheels 58, 62 for driving sheath 46 and tensile cord 48 respectively for advancing and activating end effector 38. Drive control components 112 may be included similar to drivers 60, 64 and control circuits 104, 106 discussed with reference to FIGS. 2 and 5.

Tool control module 116 is mounted to the instrument port 32 of endoscope 12 such that it is permitted to rotate about an axis through the opening of the instrument port 32. Friction drive wheels 58, 62 may also serve to fix the axial orientation of the sheath 46 and tensile cord 48 relative to tool control module 116. As tool control module 116 is rotated as indicated by arrows "E", a rotational force is transmitted through the endoscopic tool 14 causing a complementary rotation in the end effector 38 as indicated by arrows "e". Hand grips 118 may be included on the exterior of tool control module 116 to facilitate this rotation. A slip ring 120 axially surrounds a portion of tool control module 116 to provide continuous electrical communication between the electrical components on tool control module 116 and the electrical components on the endoscope body 28 as the tool control module 116 rotates. A secondary opening 122 may be maintained in longitudinal seam 50 to allow the sheath 46 and tensile cord 48 to converge on a proximal side of tool control module 116. Secondary opening 122 allows sheath 46 and tensile cord 48 to rotate together without becoming tangled when tool control module 116 is rotated.

In an exemplary use, a clinician may grasp the body 28 of endoscope 12 to position and activate end effector 38 as indicated above using control surface 30. The clinician may also adjust the orientation of end effector 38 at any time by rotating the tool control module 116. Thus, the clinician may control the position, activation and orientation of end effector 38 while maintaining his grasp on the endoscope body 28 without relying on an assistant.

Other alternatives are envisioned wherein, for example, rotation may be controlled through user interface 30. Appropriate control components 112 in conjunction with a rotational driver (not shown) may be included for such purposes.

Although the foregoing disclosure has been described in some detail by way of illustration and example, for purposes of clarity or understanding, certain changes and modifications may be practiced within the scope of the appended claims.

## Claims

1. An apparatus, comprising:
a surgical tool having a hollow tubular member;
an actuation member extending through at least a portion of the tubular member;
at least one end effector adapted to manipulate tissue and coupled to a distal end of the actuation member, the tubular member and the actuation member having a proximal actuating end where the actuation member is movable relative to the tubular member to effect movement of the at least one end effector;
a main driver operatively coupled the tubular member and adapted for causing longitudinal motion of the tubular member to effect movement thereof; and
an independent follower driver operatively coupled to the actuation member, the independent follower driver allowing selective longitudinal motion of the actuation member for moving the actuation member relative to the tubular member to actuate the at least one end effector, and for moving the actuation member together with the tubular member for positioning the at least one end effector.

2. The apparatus according to claim 1, wherein the main driver and follower driver are mounted in a tool control module, the tool control module mountable to an instrument port of an endoscope.

3. The apparatus according to claim 1 or 2, further comprising a user interface positionable on a body portion of the endoscope for accepting instructions from a user, the user interface in electrical communication with the main driver and independent follower driver such that the instructions may be transmitted from the user interface to the main driver and independent follower driver.

4. The apparatus according to claim 1, 2 or 3, wherein the tubular member comprises a sheath having a longitudinal seam such that an opening may be maintained where the actuation member may diverge from the sheath.

5. The apparatus according to any one of the preceding claims, further comprising a coupling member near a distal end of the tubular member, the coupling member for permitting selective attachment and removal of the end effector from the surgical tool.

6. The apparatus according to any one of the preceding claims, wherein the surgical tool is configured to deliver electrosurgical energy to the tissue.

7. The apparatus according to claim 2, or any one of claims 3 to 6 as dependent on claim 2, wherein the tool control module is adapted for rotation relative to the instrument port to effect a corresponding rotation in the end effector.

8. An endoscopic tool control system, comprising:
an endoscope having a proximal end adapted for handling by a clinician, a distal end adapted for positioning within a body cavity of a patient, an instrument port near the proximal end of the endoscope, the endoscope defining an instrumentation lumen therein, which extends from the instrument port to the distal end of the endoscope;
an endoscopic tool partially positionable within the instrumentation lumen, the endoscopic tool having an end effector at a distal end thereof adapted to manipulate tissue, an elongate tubular member coupled to and extending proximally from the end effector, an elongate actuation member coupled to and extending proximally from the end effector within the tubular member to a proximal end of the endoscopic tool, wherein a relative motion of the actuation member with respect to the tubular member actuates the end effector; and
a tool control module coupled to the instrument port, the tool control module including a main driver adapted to engage the tubular member and selectively impart longitudinal motion thereto, and a follower driver adapted to independently engage the actuation member and selectively impart longitudinal motion thereto.

9. The endoscopic tool control system according to claim 8, wherein the endoscope further comprises a user interface disposed on a body portion thereof, the user interface adapted to accept instructions from the clinician to effect a motion of the end effector.

10. The endoscopic tool control system according to claim 8 or 9, wherein the elongate tubular member comprises a longitudinal seam, an opening through which the actuation member may pass from an interior side of the tubular member to an exterior side of the tubular member.

11. The endoscopic tool control system according to claim 8, 9 or 10, wherein the endoscopic tool includes a coupling member for permitting selective attachment and removal of the end effector from the endoscopic tool.

12. The endoscopic tool control system according to any one of claims 8 to 11, wherein the end effector comprises an electrosurgical forceps assembly.

13. The endoscopic tool control system according to any one of claims8 to 11, wherein the end effector comprises a snare loop.
